(19) Europäisches Patentamt
European Patent Office
Office européen des brevets

(11) Veröffentlichungsnummer: **0 343 429**
**A1**

# (12) EUROPÄISCHE PATENTANMELDUNG

(21) Anmeldenummer: 89108369.3

(22) Anmeldetag: 10.05.89

(51) Int. Cl.4: C07D 401/04 , C07D 413/04 , C07D 417/04 , A61K 31/44

(30) Priorität: 16.05.88 DE 3816629

(43) Veröffentlichungstag der Anmeldung: 29.11.89 Patentblatt 89/48

(84) Benannte Vertragsstaaten: AT BE CH DE ES FR GB GR IT LI LU NL SE

(71) Anmelder: ASTA Pharma Aktiengesellschaft
Weismüllerstrasse 45
D-6000 Frankfurt am Main 1(DE)

(72) Erfinder: Engel, Jürgen, Dr.
Erlenweg 3
D-8755 Alzenau(DE)
Erfinder: Emig, Peter, Dr.
Taunusstrasse 6
D-6369 Niederdorfelden(DE)
Erfinder: Nickel, Bernd, Dr.
Alleestrasse 35
D-6109 Mühltal 6(DE)
Erfinder: Szelenyi, Istvan, Dr.
Händelstrasse 32
D-8501 Schwaig(DE)

## (54) Substituierte 3-(N-Heterocyclyl)-2,6-diaminopyridine und -N-oxide, ihre Herstellung und Verwendung als Arzneimittel.

(57) Verbindungen der allgemeinen Formel

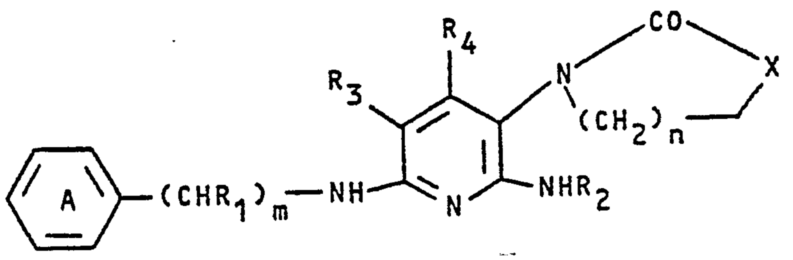

I

worin die Reste $R_1$ und $R_2$ gleich oder verschieden sind und Wasserstoff oder $C_1$-$C_6$-Alkyl bedeuten, die Reste $R_3$ und $R_4$ gleich oder verschieden sind und Wasserstoff, $C_1$-$C_6$-Alkyl, Hydroxy, $C_1$-$C_6$-Alkoxy, $C_2$-$C_6$-Alkanoyloxy, Halogen oder $C_2$-$C_6$-Alkanoyl bedeuten, X Sauerstoff, Schwefel oder die Gruppe -$NR_5$ darstellt und $R_5$ Wasserstoff, $C_1$-$C_6$-Alkyl, $C_2$-$C_6$-Alkanoyl oder Benzoyl ist, m und n jeweils die Zahlen 1, 2 oder 3 annehmen können und der Phenylrest A unsubstituiert ist oder durch Halogen, Nitro, $C_1$-$C_6$-Alkyl, Trifluormethyl, $C_3$-$C_7$-Cycloalkyl, Hydroxy, $C_1$-$C_6$-Alkoxy, $C_2$-$C_6$-Alkanoyloxy, Amino, $C_1$-$C_6$-Alkylamino, Di-$C_1$-$C_6$-alkylamino, $C_2$-$C_6$-Alkanoylamino, CN, Carboxy, $C_1$-$C_6$-Alkoxycarbonyl, Phenyl, $C_1$-$C_6$-Alkylphenyl oder Trifluormethylphenyl substituiert ist, deren Pyridin-N-oxide und deren physiologisch verträgliche Salze, Verfahren zu deren Herstellung und Arzneimittel, die solche Verbindungen als Wirkstoffe enthalten.

EP 0 343 429 A1

**Substituierte 3-(N-Heterocyclyl)-2,6-diaminopyridine und -N-oxide, ihre Herstellung und Verwendung als Arzneimittel**

Durch die belgischen Patente 698 384 und 764 362 sind Verbindungen der folgenden Formel bekannt:

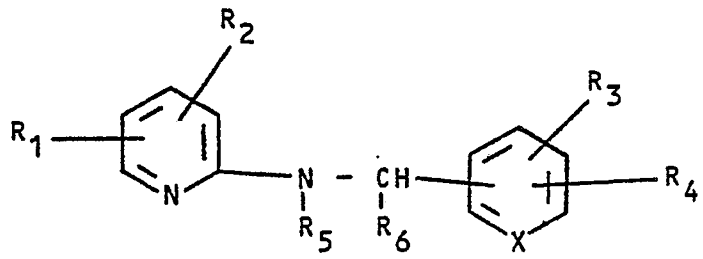

In dieser Formel bedeuten einer oder mehrere der Reste $R_1$ bis $R_4$ Aminogruppen, die acyliert oder durch niedrigmolekulare Reste alkyliert sein können, wobei diejenigen der Reste $R_1$ bis $R_4$, die keine Aminogruppen darstellen, Wasserstoff- oder Halogenatome, niedrigmolekulare Alkyl-, Trifluormethyl-, Cyan-, Rhodan-, Mercapto-, niedrigmolekulare Alkylthio-, Acylthio-, Hydroxy-, Methylendioxy-, niedrigmolekulare Alkoxy-, Acyloxy-, Nitro-, Carboxy-, Carbalkoxy- oder Carbamoylgruppen bedeuten, $R_5$ ein Wasserstoffatom oder einen Acylrest, $R_6$ ein Wasserstoffatom, eine niedrigmolekulare Alkyl- oder eine Aralkylgruppe und X ein Stickstoffatom oder die CH-Gruppe darstellt und wobei die Acylreste sich von der Kohlensäure, dem Kohlensäurehalbmorpholid, von Kohlensäuremonoestern, von vorzugsweise substituierten Benzoesäuren und Pyridincarbonsäuren oder von gesättigten oder ungesättigten, gegebenenfalls durch einen Morpholinorest substituierten niedrigmolekularen aliphatischen Mono- oder Dicarbonsäuren ableiten.
Für diese Verbindungen wird eine antiphlogistische und analgetische Wirksamkeit angegeben, wobei es sich bei der analgetischen Wirkung um eine spezifisch zentralanalgetische Wirkung handelt, das heißt eine Wirkung auf die Nervenzellen des Rückenmarks und/oder die Nervenzellen des Gehirns.

Die Erfindung betrifft neue Triamino-Pyridine mit einem heterocyclischen Rest in 3-Stellung gemäß der Formel I. Diese Verbindungen haben verbesserte Eigenschaften.

Die erfindungsgemäßen Verbindungen sind pharmakologisch wirksam. Die erfindungsgemäßen Verbindungen besitzen eine ausgeprägte antikonvulsive und antiepileptische Wirkung.
Von besonderem Vorteil ist dabei die hohe therapeutische Breite, wobei insbesondere Nebenwirkungen (wie zum Beispiel Sedierung, Ataxie, alkoholverstärkende Wirkung) in den antikonvulsiv-, antiepileptisch- und peripher analgetisch wirksamen Dosen äußerst gering sind.
Einige Verbindungen besitzen darüber hinaus noch eine spezifische peripher analgetische Wirkung (Wirkung auf das periphere Nervensystem) sowie eine antiphlogistische Wirkung.

Der Erfindung liegt also die Aufgabe zugrunde, Verbindungen mit günstigen pharmakologischen Eigenschaften zur Verfügung zu stellen, die beispielsweise als antikonvulsiv wirkende Arzneimittel verwertbar sind.

Die folgenden Erläuterungen stellen bevorzugte Aufführungen dar:

Die in der Formel I vorkommenden Alkylgruppen, Alkoxygruppen oder Alkanoylgruppen können gerade oder verzweigt sein. Dasselbe gilt auch für Alkyl- und Alkoxygruppen, falls diese Bestandteil anderer zusammengesetzter Gruppen sind (zum Beispiel in Form einer Monoalkyl- oder Dialkylaminogruppe, Alkanoylaminogruppe, Alkanoyloxygruppe, Alkoxycarbonylgruppe und ähnlichen Gruppen). Bei den Halogenatomen handelt es sich um Chlor, Brom oder Fluor, insbesondere Chlor und Fluor. Die Alkyl- und Alkoxygruppen als solche oder als Bestandteil von anderen zusammengesetzten Gruppen bestehen insbesondere aus 1-4 C-Atomen, vorzugsweise 1 oder 2 C-Atomen. Alkanoylgruppen, Alkanoyloxygruppen oder Alkanoylaminogruppen bestehen insbesondere aus 2-4, vorzugsweise 2-3 C-Atomen.

Die $C_3$-$C_7$-Cycloalkylgruppe besteht insbesondere aus 5 bis 6 C-Atomen.
X bedeutet vorzugsweise Sauerstoff oder die Gruppe $-NR_5$.
Besonders günstige Eigenschaften weisen solche Verbindungen der Formel I auf, worin X Sauerstoff, die Reste $R_1$, $R_2$, $R_3$ und $R_4$ Wasserstoff und m die Zahl 1 ist, n 1 oder 2 ist und der Phenylring A vorzugsweise einen der angegebenen Substituenten enthält, wobei ein solcher Substituent vorzugsweise ein Halogenatom (beispielsweise Fluor, Chlor) oder die Trifluormethylgruppe ist. Falls der Phenylring A einen Substituenten enthält, befindet sich dieser vorzugsweise in 4-Stellung, Substitution in 2-und/oder 3-Stellung

ist jedoch ebenfalls möglich. Der Phenylring A kann aber auch 2, 3, 4 oder 5 der angegebenen Substituenten enthalten, wobei diese gleich oder verschieden sein können.
Falls der Phenylring A durch einen Trifluormethylphenylrest substituiert ist, handelt es sich insbesondere um den 4-Trifluormethyl-phenylrest. Das gleiche gilt für den Fall, daß der Phenylring A durch einen $C_1$-$C_6$-Alkylphenylrest substituiert ist. Der $C_1$-$C_6$-Alkylrest ist hier vorzugsweise Methyl oder Ethyl.

Das Verfahren zur Herstellung von Verbindungen der Formel I aus Verbindungen der Formel II wird in einem Lösungs- oder Dispergiermittel oder auch ohne Lösungsmittel bei Temperaturen zwischen 0 und 220° C durchgeführt. Falls ein Lösungs- oder Dispergiermittel verwendet wird, kommmt vorzugsweise ein Temperaturbereich zwischen 0 und 100° C, insbesondere 5 und 60° C oder auch 10 und 50° C in Frage. Ohne Lösungsmittel erfolgt die Reaktion in der Schmelze beispielsweise zwischen 150 und 220° C, vorzugsweise 160 - 190° C.

Falls n = 2 ist, wird das Verfahren im allgemeinen bei Temperaturen zwischen 20 und 70° C, vorzugsweise 40 - 50° C, falls n = 1 ist, im allgemeinen bei Temperaturen zwischen 10 und 40, vorzugsweise 10 - 30, insbesondere 20 - 25° C durchgeführt.

Bei der veresterten Hydroxygruppe (Bedeutung von Y) handelt es sich um reaktionsfähige Ester. Ein reaktionsfähiger Ester ist dabei derjenige einer starken organischen oder anorganischen Säure, wie vor allem einer Halogenwasserstoffsäure, zum Beispiel der Chlor-, Brom- oder Jodwasserstoffsäure, oder einer Sulfonsäure, wie einer Aryl- oder $C_1$-$C_6$-Alkylsulfonsäure, zum Beispiel von niederen Alkylbenzolsulfonsäuren (p-Toluolsulfonsäure). Insbesondere ist Y Halogen (CL, Br, J).

Als Lösungs- beziehungsweise Dispergiermittel kommen zum Beispiel in Frage: niedere aliphatische Alkohole (1-6 C-Atome wie Methanol, Ethanol, Propanol, Isopropanol, Butanol), niedere aliphatische Ether (Diethylether, Diisopropylether), aromatische Kohlenwasserstoffe (Benzol, Toluol, Xylol), cyclische Ether (Dioxan, Tetrahydrofuran), Ester von niederen aliphatischen Carbonsäuren mit niederen aliphatischen Alkoholen, Amide und N-alkyl-substituierte Amide von aliphatischen $C_1$-$C_4$-Carbonsäuren (Dimethyl formamid, Dimethylacetamid), $C_1$-$C_6$-Dialkylsulfone (Dimethylsulfon, Tetramethylensulfon), $C_1$-$C_6$-Dialkylsulfoxide (Dimethylsulfoxid) sowie weitere aprotische Mittel wie N-Methylpyrrolidon, Tetramethylharnstoff, Hexamethylphosphorsäuretriamid, Acetonitril.
Die einzelnen Alkylreste der oben angegebenen Lösungsmittel enthalten beispielsweise 1-6, insbesondere 1-4 Kohlenstoffatome.
Das Verfahren wird gegebenenfalls in Gegenwart von Kondensationsmitteln durchgeführt. Als derartige Kondensationsmittel kommen zum Beispiel in Frage: anorganische Kondensationsmittel wie Ammoniak, Alkali-oder Erdalkalihydroxide (NaOH, KOH), Alkalihydride, Alkaliamide, Alkali- oder Erdalkalicarbonate oder organische Basen wie Pyridin, tertiäre Amine, Piperidin, Alkalialkoholate, Alkaliacetate oder auch Triethylphosphat. Bei den Alkalimetallen handelt es sich insbesondere um Natrium oder Kalium. Es kann auch unter Phasen-Transfer-Bedingungen (das heißt unter Zusatz eines oder mehrerer langkettiger Amine wie einem Benzyltributylammonium-halogenid, einem Tetrabutyl-ammonium-halogenid oder Benzyl-triphenylphosphoniumchlorid) gearbeitet werden.
Es kann auch eine Ausgangskomponente der Formel II verwendet werden, die anstelle des Restes $R_2$ eine übliche Amino-Schutzgruppe enthält, welche nach Beendigung der Reaktion leicht solvolytisch oder hydrierend abspaltbar ist.
Vorzugsweise wird die Ausgangssubstanz II in Form ihres Salzes mit einer anorganischen Säure (zum Beispiel als Hydrochlorid) eingesetzt.

Ausgangsstoffe der Formel II können beispielsweise wie folgt erhalten werden:

In Verbindungen der Formel

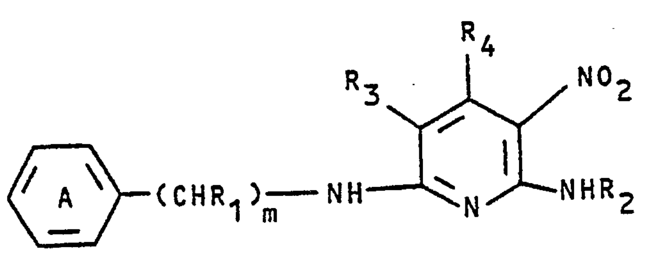

III

die bekannt sind beziehungsweise in analoger Weise wie die entsprechenden bekannten Verbindungen erhalten werden können (siehe belgische Patente 698 384, 764 362, 736 139; DOS 33 37 593), wird die Nitrogruppe in der 3-Stellung in hierfür bekannter Weise reduziert (zum Beispiel wie in den zuvor angegebenen Druckschriften für die Reduktion der 3-ständigen Nitrogruppe eines Pyridinringes angegeben

ist) und die so erhaltene Verbindung III mit der 3-ständigen Aminogruppe zum Beispiel mit einer Verbindung der Formel

$Hal^1 - CO - X(CH_2)_n - Hal^2$ IV

oder der Formel $Hal^1 - CO - X(CH_2)_n - OH$ V

worin X und n die angegebenen Bedeutungen haben und $Hal^1$ und $Hal^2$ gleich oder verschieden sind und Chlor, Brom oder Jod bedeuten, in hierfür üblicher Weise umsetzt.

Anstelle der Verbindungen IV und V können auch entsprechende äquivalente Verbindungen verwendet werden, die geeignet sind ein Wasserstoffatom der durch die Reduktion erhaltenen 3-ständigen Aminogruppe durch die Acylgruppe $-CO-X-(CH_2)_n-Y$ zu ersetzen. Zweckmäßig erfolgt die Acylierung der 3-ständigen Aminogruppe sofort im Anschluß an die Reduktion der Nitrogruppe in der hier vorliegenden Reaktionsmischung. Diese Umsetzung kann zum Beispiel bei Temperaturen zwischen - 10 und 100° C vorzugsweise O und 60° C erfolgen.

Als Suspensions- oder Lösungsmittel kommen für diese Umsetzung zum Beispiel in Frage: Gesättigte alicyclische und cyclische Ether (Dioxan, Tetrahydrofuran, niedere Dialkylether wie Diethylether, Diisopropylether), niedere Alkanole wie Ethanol, Isopropanol, Butanol, niedere aliphatische Ketone (Aceton, Methylethylketon), niedere aliphatische Kohlenwasserstoffe oder Halogenkohlenwasserstoffe (Methylenchlorid, Chloroform, 1,2-Dichlorethan), aromatische Kohlenwasserstoffe (Benzol, Toluol, Xylol), niedere Dialkylamide von niederen gesättigten aliphatischen Carbonsäuren (Dimethylformamid, Dimethylacetamid), Tetramethylharnstoff, N-Methylpyrrolidon, Dimethylsulfoxid beziehungsweise Mischungen dieser Mittel.

Im allgemeinen werden die Reaktionskomponenten in molaren Mengen umgesetzt. Gegebenenfalls kann es jedoch zweckmäßig sein, eine Reaktionskomponente in leichtem Überschuß einzusetzen. Gegebenenfalls kann die Umsetzung auch in Gegenwart von basischen beziehungsweise säurebindenden Mitteln, wie Alkalicarbonaten (Pottasche, Soda), Alkalihydrogencarbonaten, Alkaliacetaten, Alkalihydroxyden, tertiären Aminen (beispielsweise Triethylamin) oder basischen Ionenaustauschern durchgeführt werden. Letzteres gilt insbesondere, wenn Halogenameisensäureester-Derivate eingesetzt werden. Das Verfahren kann jedoch auch ohne basisches Kondensationsmittel beziehungsweise Säurefänger durchgeführt werden, indem die Verbindung II als Säurefänger dient und somit das HCl-Salz der gewünschten Verbindung entsteht.

Falls die Ausgangsstoffe der Formel III nicht bekannt sind, können solche Ausgangsstoffe beispielsweise durch Umsetzung von 3-Nitropyridinen der Formel

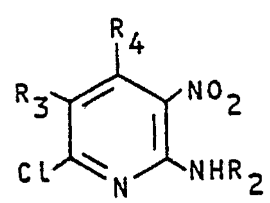

mit Aminen der Formel

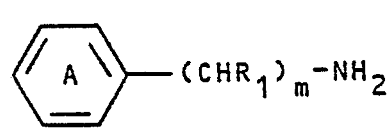

mit oder ohne Lösungsmittel bei Temperaturen zwischen 0 bis 200° C gegebenenfalls in Gegenwart eines zusätzlichen Salzsäureakzeptors analog der in den belgischen Patentschriften 698 384, 764 362 oder 736 139 beschriebenen Weise oder gemäß der deutschen Patentschrift 1 795 797 erhalten werden. Die zuvor erwähnten 3-Nitropyridine können zum Beispiel aus den entsprechenden 2,6-Dichlor-pyridinen (mit den Resten $R_3$ und $R_4$) durch hierfür übliche Nitrierung und anschließender Aminolyse mit Aminen $R_2-NH_2$ - (Ersatz des Cl in 2-Stellung) erhalten werden.

Die eingesetzten 2,6-Dichloropyridine können beispielsweise durch Behandeln von Pyridinen der Formel

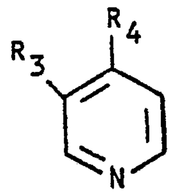

mit Chlorierungsmitteln (Thionylchlorid, Sulfurylchlorid, Phosphortrichlorid, Phosphoroxytrichlorid, Phosphorpentachlorid, HCl) in der üblichen Weise erhalten werden. Vorhandene freie Hydroxygruppen werden hierbei gegebenenfalls durch übliche Schutzgruppen geschützt, die gegebenenfalls nach der Chlorierung abgespalten werden.

Ausgangsverbindungen der Formel II, worin Y die veresterte Hydroxygruppe ist, können auch aus solchen Ausgangsstoffen der Formel II erhalten werden, worin Y eine Hydroxygruppe ist durch Umsetzung mit Thionylhalogeniden (Chloriden, Bromiden, Jodiden) oder äquivalenten Mitteln in Halogenkohlenwasserstoffen (Chloroform) oder aromatischen Kohlenwasserstoffen (Benzol) oder in Pyridin bei Temperaturen zwischen 20 und 150° C (vorzugsweise Siedetemperatur des verwendeten Lösungsmittels). Ausgangsstoffe der Formel II, worin Y eine Hydroxygruppe ist, die durch eine andere Säure wie Halogenwasserstoff verestert ist (wo Y also zum Beispiel eine Alkylsulfonyloxygruppe oder eine Arylsulfonyloxygruppe ist), können zum Beispiel aus den entsprechenden Hydroxyverbindungen (Y = OH) durch Umsetzung mit $C_1$-$C_6$-Alkyl-sulfonsäurechloriden oder den entsprechenden Arylsulfonsäure chloriden in hierfür üblichen inerten Lösungsmitteln (Benzol, Toluol, Xylol, Chloroform, Methylenchlorid, Dioxan) bei Temperaturen zwischen 20 - 150° C erhalten werden. Zweckmäßig wird hierbei in Gegenwart einer säurebindenden Substanz (zum Beispiel tertiäre Amine wie Triethylamin) gearbeitet.

Falls $R_2$ Wasserstoff ist, kann die Aminogruppe durch eine leicht abspaltbare Schutzgruppe geschützt werden; dasselbe gilt analog für weitere gegebenenfalls vorhandene Aminogruppen und/oder Hydroxygruppen.

Verfahrensprodukte der Formel I, die freie Hydroxygruppen, Aminogruppen enthalten oder wo X die Gruppe -$NR_5$ mit $R_5$ = Wasserstoff ist, können an diesen Gruppen acyliert werden. Dabei handelt es sich um die Einführung von $C_2$-$C_6$-Alkanoylgruppen, $C_1$-$C_6$-Alkoxycarbonylgruppen oder auch um Einführung einer Benzoylgruppe (falls X beispielsweise die Gruppe -$NR_5$ darstellt und $R_5$ Benzoyl ist). Diese Acylierung kann in inerten Lösungs- beziehungsweise Suspensionsmitteln bei Temperaturen zwischen 0 bis 200° C, vorzugsweise 20 bis 150° C, erfolgen. Als Lösungs- oder Dispergiermittel kommen beispielsweise in Betracht: aromatische Kohlenwasserstoffe wie zum Beispiel Benzol, Toluol, Xylol; aliphatische Ketone wie zum Beispiel Aceton, Methylethylketon; halogenierte Kohlenwasserstoffe wie zum Beispiel Chloroform, Tetrachlorkohlenstoff, Chlorbenzol, Methylenchlorid; aliphatische Ether wie zum Beispiel Butylether; cyclische Ether wie zum Beispiel Tetrahydrofuran, Dioxan; Sulfoxyde wie zum Beispiel Dimethylsulfoxyd; tertiäre Säureamide wie zum Beispiel Dimethylformamid, Dimethylacetamid, N-Methypyrrolidon, Hexamethylphosphorsäuretriamid; aliphathische Alkohole wie Methanol, Ethanol, Isopropanol, Amylalkohol, tert.-Butanol, cycloaliphatische Kohlenwasserstoffe wie Cyclohexan und ähnliche. Auch wässrige Mischungen der genannten Lösungsmittel können gegebenenfalls verwendet werden. Häufig arbeitet man bei der Rückflußtemperatur der verwendeten Lösungs- beziehungsweise Dispergiermittel.

Als Acylierungsmittel kommen in Betracht: Ketene sowie Säurehalogenide (Chloride, Bromide, Jodide), Säureanhydride oder Säureester aliphatischer Carbonsäuren mit 2-6 C-Atomen, beziehungsweise $C_1$-$C_6$-Alkoxycarbonylhalogenide (Chloride, Bromide).

Als Lösungs- beziehungsweise Suspensionsmittel kommen hierfür beispielsweise in Betracht: Wasser, niedere aliphatische Alkohole, cyclische Äther wie Dioxan oder Tetrahydrofuran, aliphatische Äther, Dimethylformamid und so weiter sowie Mischungen dieser Mittel.

Gegebenenfalls erfolgt Zusatz von säurebindenden Mitteln wie Alkalicarbonaten, Alkalihydroxyden, Alkalialkoholaten oder eines tertiären Amins, zum Beispiel Triäthylamin oder Pyridin. Pyridin kann gleichzeitig auch als Lösungsmittel verwendet werden. Bei den oben genannten Estern handelt es sich insbesondere um solche der oben genannten Carbonsäuren mit niederen aliphatischen Alkoholen. Bei der Acylierung kann man auch so vorgehen, daß man erst von der umzusetzenden Verbindung eine Alkaliverbindung herstellt, indem man sie in einem inerten Lösungsmittel wie Dioxan, Dimethylformamid, Benzol oder Toluol mit einem Alkalimetall, Alkalihydriden oder Alkaliamiden (insbesondere Natrium oder Natriumverbindungen) bei Temperaturen zwischen 0 und 150° C umsetzt und dann das acylierende Agens zufügt.

Anstelle der angeführten Acylierungsmittel können auch andere in der Chemie gebräuchliche chemischäquivalente Mittel verwendet werden (siehe zum Beispiel auch L.F. und Mary Fieser "Reagents for Organic

Syntheses", John Wiley and Sons, Inc., New York, 1967, Vol. 1, Seiten 1303-4 und Vol. 2, Seite 471).

Diese Acylgruppen in den Verbindungen der Formel I können solvolytisch wieder abgespalten werden. Diese solvolytische Abspaltung erfolgt beispielsweise durch Verseifung mit verdünnten Säuren oder mittels basischer Substanzen (Pottasche, Soda, wässrige Alkalilösungen, alkoholische Alkalilösungen, $NH_3$) bei Temperaturen zwischen 10 und 150° C, insbesondere 20-100° C.

Als Lösungs- beziehungsweise Suspensionsmittel kommen hierfür beispielsweise in Betracht: Wasser, niedere aliphatische Alkohole, cyclische Äther wie Dioxan oder Tetrahydrofuran, aliphatische Äther, Dimethylformamid und so weiter sowie Mischungen dieser Mittel.

Die Überführung von Verbindungen der Formel I in die Pyridin-N-oxide kann beispielsweise in Lösungsmitteln wie niederen Alkoholen (Methanol, Ethanol), Chloroform oder anderen Chlorkohlenwasserstoffen, Benzol, Toluol, Aceton, verdünnter Essigsäure, Essigsäureäthylester oder niederen aliphatischen Säureanhydriden (zum Beispiel Acetanhydrid) mit Wasserstoffperoxid, einer üblichen aliphatischen oder aromatischen Persäure (Peressigsäure, Benzopersäure, m-Chlorbenzopersäure) oder anderen Monosubstitutionsprodukten des Wasserstoffperoxids wie Alkaliperoxiden oder Alkylperoxiden (zum Beispiel tert.-Butylperoxid) bei Temperaturen zwischen 0 und 150° C, vorzugsweise 0 bis 100° C, durchgeführt werden.

Die rein erhaltenen Verbindungen der allgem. Formel I liegen als Basen vor und können gegebenenfalls durch Umsetzung mit Säuren in therapeutisch verwendbare Salze überführt werden.

Als solche Säuren seien beispielsweise genannt: Halogenwasserstoffsäuren, Schwefelsäure, Phosphorsäuren, Salpetersäure, Perchlorsäure, organische Mono-, Di- oder Tricarbonsäuren der aliphatischen, alicyclischen, aromatischen oder heterocyclischen Reihe sowie Sulfonsäuren. Beispiele hierfür sind: Ameisen-, Essig-, Propion-, Bernstein-, Glykol-, Milch-, Äpfel-, Wein-, Zitronen-, Ascorbin-, Malein-, Fumar-, Hydroxymalein-, Glucon- oder Brenztraubensäure;
Phenylessig-, Benzoe-, p-Aminosalicylsäure, Embonsäure, Methansulfon-, Ethansulfon-, Hydroxyethansulfon-, Ethylensulfonsäure; Halogenbenzolsulfon-, Toluolsulfon-, Naphthalinsulfonsäure oder Sulfanilsäure oder auch 8-Chlor-theophyllin.

Die erfindungsgemäßen Verbindungen zeigen zum Beispiel im Maximalen Elektroschock-Test eine gute antiepileptisch Wirkung.
Beispielsweise wird bei obengenannter Versuchsmethode bei einer Dosis von 56 mg/Körpergewicht Maus eine antiepileptische Wirkung bei 50 % der Tiere erhalten.

Die niedrigste, bereits wirksame Dosis in dem obenangegebenen Tierversuch ist beispielsweise
20 mg/kg oral
5 mg/kg intravenös

Als allgemeiner Dosisbereich für die Wirkung (Tierversuch wie oben) kommt beispielsweise in Frage:
20 - 100 mg/kg oral, insbesondere 40 - 80 mg/kg Maus
5 - 25 mg/kg intravenös, insbesondere
10 - 20 mg/kg Maus

Die Wirkungsrichtung der erfindungsgemäßen Verbindungen ist mit der Wirkung des bekannten Arzneimittelwirkstoffs Phenytoin beziehungsweise Carbamazepin vergleichbar. Die erfindungsgemäßen Verbindungen besitzen hingegen zum Beispiel eine größere therapeutische Breite.

Indikationen für die die erfindungsgemäßen Verbindungen in Betracht kommen können: Epilepsie

Die pharmazeutischen Zubereitungen enthalten im allgemeinen zwischen 20 bis 200 vorzugsweise 50 bis 100 mg der erfindungsgemäßen aktiven Komponente(n).

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspensionen und Emulsionen. Bevorzugte Ansendungsformen sind Tabletten, die zwischen 50 und 100 mg oder Lösungen, die zwischen 1,5 bis 5 Gewichtsprozent an aktiver Substanz enthalten.

Die Einzeldosis der erfindungsgemäßen aktiven Komponenten kann beispielsweise liegen

a) bei oralen Anrzneiformen zwischen 20 und 400 mg, vorzugsweise 100 mg

b) bei parenteralen Arzneiformen (zum Beispiel intravenös, intramuskulär) zwischen 5 und 40 mg, vorzugsweise 20 mg

c) bei Arzneiformen zur rektalen Applikation zwischen 20 und 400 mg, vorzugsweise 100 mg

-Diese Dosen sind jeweils bezogen auf die freie Base-

Beispielweise können 3 mal täglich 1 bis 2 Tabletten mit einem Gehalt von 50 bis 100 mg wirksamer Substanz oder zum Beispiel bei intravenöser Injektion 1 bis 2 mal täglich eine Ampulle von 2 bis 5 ml Inhalt mit 10 bis 20 mg Substanz empfohlen werden. Bei oraler Verabreichung ist die minimale tägliche Dosis beispielsweise 150; die maximale tägliche Dosis bei oraler Verabreichung soll nicht über 600 liegen.

Für die Behandlung von Hunden und Katzen liegt die orale Einzeldosis im allgemeinen zwischen ungefähr 10 und 20 mg/kg Körpergewicht; die parenterale Dosis ungefähr zwischen 2 und 10 mg/kg Körpergewicht.

Die akute Toxizität der erfindungsgemäßen Verbindungen an der Maus (ausgedrückt durch die LD 50 mg/kg; Methode nach Miller und Tainter: Proc. Soc. Exper. Biol. a. Med. 57 (1944) 261) liegt beispielsweise bei oraler Applikation zwischen 1000 und 1500 mg/kg (beziehungsweise oberhalb 1200 mg/kg.

Die Arzneimittel können in der Humanmedizin, der Veterinärmedizin sowie in der Landwirtschaft allein oder im Gemisch mit anderen pharmakologisch aktiven Stoffen verwendet werden.

## Beispiel 1

2-Amino-3-(oxazolidin-2-on-3-yl)-6-(4-fluorbenzylamino)-pyridin

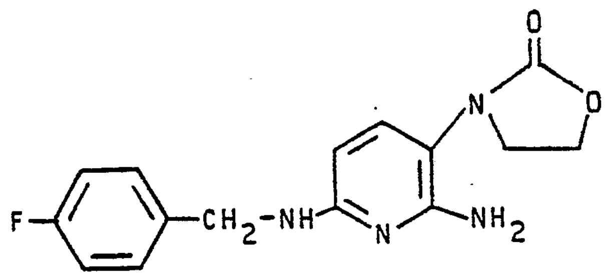

Eine Suspension von 50 g (0,12 Mol) 2-Amino-3-[(2-bromethoxy)-carbonylamino]-6-(4-fluor-benzylamino)-pyridin-Hydrochlorid in 200 ml Methanol wird zu 250 ml 10 %igem ethanolischen Ammoniak gegeben und die Mischung insgesamt 24 Stunden bei Raumtemperatur unter Argon gerührt (hierbei werden nach Ablauf von 12 Stunden nochmals 100 ml 10 %iges ethanolisches Ammoniak zugesetzt). Die entstandene kristalline Verbindung wird abgesaugt, mit eisgekühltem Isopropanol gewaschen und im Vakuum bei Raumtemperatur getrocknet.
F.: 176 °C
Ausbeute: 21,6 g

Die Herstellung der Verbindung kann auch wie folgt durchgeführt werden:

Zu der Suspension von 50 g 2-Amino-3-[(2-bromethoxy)-carbonylamino]-6-(4-fluorbenzylamino)-pyridin-hydrochlorid in 200 ml Methanol wird 2n Natronlauge bis pH 10,5 zugegeben und nach ca. 30 Minuten durch erneute Zugabe von 2n Natronlauge der pH wiederum auf pH 10, 5 eingestellt.

Zur Bildung des Hydrochlorids werden 21,6 g 2-Amino-3-(oxazolidin-2-on-3-yl)-6-(4-fluor-benzylamino)-pyridin unter Argon-Atmosphäre in kaltem Aceton gelöst und mit 5,5 N isopropanolischer Salzsäure auf pH 4 eingestellt. Nach circa 15 Minuten fällt das kristalline 2-Amino-3-(oxazolidin-2-on-3-yl)-6-(4-fluor-benzylamino)-pyridin-Hydrochlorid aus. Man saugt ab, wäscht mit eisgekühltem Aceton nach und trocknet im Vakuum bei Raumtemperatur.
F.: 220 °C
Ausbeute: 23,4 g (97% der Theorie)

Die verwendete Ausgangssubstanz wird beispielsweise wie folgt hergestellt:

20 g 2-Amino-3-nitro-6-(4-fluor-benzylamino)-pyridin werden mit 15,6 g Raney-Nickel und 15,6 g Magnesiumsulfat (wasserfrei) in 215 ml Dioxan suspendiert und bei 5 bar und 60 °C 2,5 Stunden hydriert. Man kühlt auf 25 °C, saugt das Raney-Nickel und Magnesiumsulfat ab und versetzt mit 17,1 g Chlorameisensäure-2-bromethylester. Das nach ungefähr 1 Stunde ausgefallene, kristalline 2-Amino-3-[(2-bromethoxy)-carbonylamino]-6-(4-fluor-benzylamino)-pyridin-Hydrochlorid wird mit wenig eisgekühltem Isopropanol und mit Ether gewaschen und im Vakuum bei Raumtemperatur getrocknet.
F. des Hydrochlorids: 216 °C.
Ausbeute: 25 g

Analog Beispiel 1 werden aus den entsprechenden Ausgangsstoffen II die in Tabelle 1 aufgeführten Verbindungen der folgenden Formel hergestellt

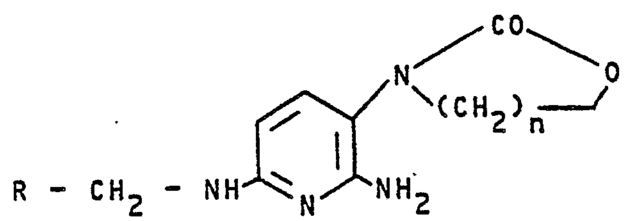

Tabelle 1

Menge ethanolisches Ammoniak (insgesamt jeweils 60 ml; Menge Lösungsmittel (Methanol) jeweils 35 ml).

| Beispiel-Nr. | R | n | Ausbeute % | F °C (Hydrochlorid) | Ausgangsstoff II (als HCl-Salz) Mol | F °C |
|---|---|---|---|---|---|---|
| 2 | 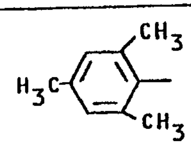 | 1 | 65,3 | 210 | 0,015 | 215 (Zers.) |
| 3 | 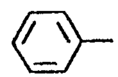 | 1 | 63,3 | 186 | 0,037 | 219 (Zers.) |
| 4 | 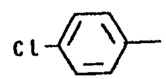 | 1 | 73,7 | 201 | 0,029 | 205 (Zers.) |
| 5 | 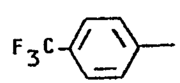 | 1 | 77,3 | 221 | 0,017 | 206 (Zers.) |
| 6 | 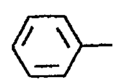 | 2 | 64,2 | 237 | 0,021 | 220 (Zers.) |
| 7 | 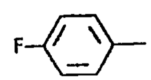 | 2 | 68,5 | 254 | 0,02 | 220 (Zers.) |

Zers. = Zersetzung

Beispiele für galenische Zubereitungen

Kapseln mit 100 mg Wirkstoff

10 kg Verbindung gemäß Beispiel 1 (Hydrochlorid) werden in einer Wirbelschicht-Sprühgranulationsapparatur mit einer Lösung aus 0,25 kg Gelatine in 2,25 kg Wasser in bekannter Weise granuliert. Nach Zumischen von 0,80 kg Maisstärke, 0,1 kg Magnesiumstearat und 0,05 kg hochdispersem Siliciumdioxid wird die Mischung in einer Füllmenge von jeweils 112 mg in Hartgelatinekapseln der Größe 3 gefüllt.

Eine Kapsel enthält 100 mg Wirksubstanz.

Suppositorien mit 150 mg Wirkstoff

1,5 kg Verbindung gemäß Beispiel 1 (Hydrochlorid) werden in 19 kg geschmolzenem Hartfett* suspendiert. Nach Homogenisierung wird die Suspension in üblicher Weise in Hohlzellen von 2,3 ml ausgegossen und abgekühlt.

(*) Hartfett ist ein Gemisch von Mono-, Di- und Tri-glyceriden der gesättigten Fettsäuren von $C_{10}H_{20}O_2$ bis $C_{18}H_{36}O_2$.

Ein Suppositorium vom Gewicht 2,05 g enthält 150 mg Wirkstoff.

## Ansprüche

1. Verbindungen der allgemeinen Formel

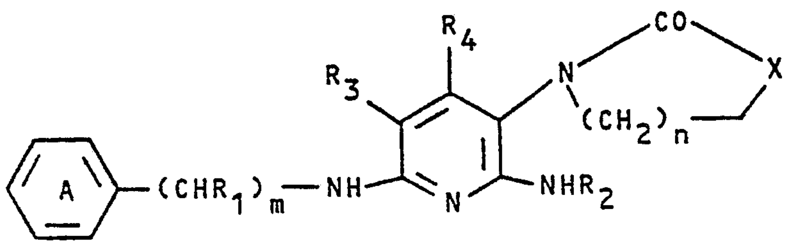

I

worin die Reste $R_1$ und $R_2$ gleich oder verschieden sind und Wasserstoff oder $C_1$-$C_6$-Alkyl bedeuten, die Reste $R_3$ und $R_4$ gleich oder verschieden sind und Wasserstoff, $C_1$-$C_6$-Alkyl, Hydroxy, $C_1$-$C_6$-Alkoxy, $C_2$-$C_6$-Alkanoyloxy, Halogen oder $C_2$-$C_6$-Alkanoyl bedeuten, X Sauerstoff, Schwefel oder die Gruppe -$NR_5$ darstellt und $R_5$ Wasserstoff, $C_1$-$C_6$-Alkyl, $C_2$-$C_6$-Alkanoyl oder Benzoyl ist, m und n jeweils die Zahlen 1, 2 oder 3 annehmen können und der Phenylrest A unsubstituiert ist oder durch Halogen, Nitro, $C_1$-$C_6$-Alkyl, Trifluormethyl, $C_3$-$C_7$-Cycloalkyl, Hydroxy, $C_1$-$C_6$-Alkoxy, $C_2$-$C_6$-Alkanoyloxy, Amino, $C_1$-$C_6$-Alkylamino, Di-$C_1$-$C_6$-alkylamino, $C_2$-$C_6$-Alkanoylamino, CN, Carboxy, $C_1$-$C_6$-Alkoxycarbonyl, Phenyl, $C_1$-$C_6$-Alkylphenyl oder Trifluormethylphenyl substituiert ist, deren Pyridin-N-oxide und deren physiologisch verträgliche Salze.

2. Verfahren zur Herstellung von Verbindungen der allgemeinen Formel

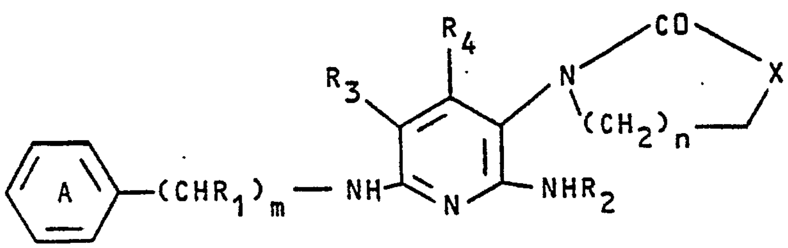

I

worin die Reste $R_1$ und $R_2$ gleich oder verschieden sind und Wasserstoff oder $C_1$-$C_6$-Alkyl bedeuten, die Reste $R_3$ und $R_4$ gleich oder verschieden sind und Wasserstoff, $C_1$-$C_6$-Alkyl, Hydroxy, $C_1$-$C_6$-Alkoxy, $C_2$-$C_6$-Alkanoyloxy, Halogen oder $C_2$-$C_6$-Alkanoyl bedeuten, X Sauerstoff, Schwefel oder die Gruppe -$NR_5$ darstellt und $R_5$ Wasserstoff, $C_1$-$C_6$-Alkyl, $C_2$-$C_6$-Alkanoyl oder Benzoyl ist, m und n jeweils die Zahlen 1, 2 oder 3 annehmen können und der Phenylrest A unsubstituiert ist oder durch Halogen, Nitro, $C_1$-$C_6$-Alkyl, Trifluormethyl, $C_3$-$C_7$-Cycloalkyl, Hydroxy, $C_1$-$C_6$-Alkoxy, $C_2$-$C_6$-Alkanoyloxy, Amino, $C_1$-$C_6$-Alkylamino, Di-$C_1$-$C_6$-alkylamino, $C_2$-$C_6$-Alkanoylamino, CN, Carboxy, $C_1$-$C_6$-Alkoxycarbonyl, Phenyl, $C_1$-$C_6$-Alkylphenyl oder Trifluormethylphenyl substituiert ist, deren Pyridin-N-oxiden und deren physiologisch verträglichen

Salzen, dadurch gekennzeichnet, daß man eine Verbindung der Formel

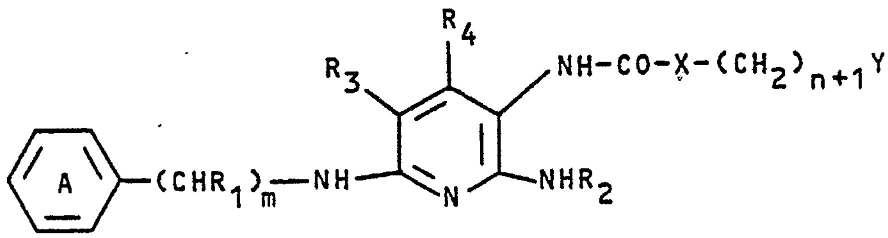

II

worin $R_1$, $R_2$, $R_3$, $R_4$ X und n die angegebenen Bedeutungen haben und Y eine durch eine starke anorganische oder organische Säure veresterte Hydroxygruppe bedeutet, unter Abspaltung von HY cyclisiert, gegebenenfalls vorhandene freie Hydroxygruppen und/oder Aminogruppen acyliert, und/oder zum entsprechenden Pyridin-N-oxid oxydiert und die erhaltenen Verbindungen gegebenenfalls in ihre Salze überführt.

3. Verbindungen der Formel I zur Anwendung als therapeutische Wirkstoffe.

4. Arzneimittel, enthaltend eine Verbindung der allgemeinen Formel I neben üblichen Träger- und/oder Verdünndungs- beziehungsweise Hilfsstoffen.

5. Verfahren zur Herstellung eines Arzneimittels,
dadurch gekennzeichnet,
daß eine Verbindung der allgemeinen Formel I mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

6. Verwendung von Verbindungen der allgemeinen Formel I zur Herstellung von Arzneimitteln.

Europäisches Patentamt

# EUROPÄISCHER RECHERCHENBERICHT

Nummer der Anmeldung

EP 89108369.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 3 337 593 (DEGUSSA) * Anspruch 1 * -- | 1,6 | C 07 D 401/04<br>C 07 D 413/04<br>C 07 D 417/03<br>A 61 K 31/44 |
| A | WO - A2 - 87/01 706 (UPJOHN) * Anspruch 28 * -- | 1 | |
| A | EP - A1 - 0 263 213 (UPJOHN) * Preparation A1-A50 * -- | 1 | |
| A | CHEMICAL ABSTRACTS, Band 93, Nr. 9, 1. September 1980, Columbus, Ohio, USA VON BEBENBURG, W. et al. "Substituted polyaminopyridines." Seite 628, Spalte 2, Zusammenfassung Nr. 95 098v & Chem.Ztg. 1979, 103(2), 387-99 -- | 1,3-6 | RECHERCHIERTE SACHGEBIETE (Int. Cl.4) |
| A | CHEMICAL ABSTRACTS, Band 98, Nr. 3, 17. Jänner 1983, Columbus, Ohio, USA TEMPLE,CAROLL JR. et al. "1,2-Dihydropyrido/3,4-b/pyrazines: structure-activity relationships." Seite 16, Spalte 1, Zusammenfassung Nr. 11 106p & J.Med.Chem. 1988, 26(1), 91-5 ---- | 1 | C 07 D 401/00<br>C 07 D 413/00<br>C 07 D 417/00 |

Der vorliegende Recherchenbericht wurde für alle Patentansprüche erstellt.

| Recherchenort | Abschlußdatum der Recherche | Prüfer |
|---|---|---|
| WIEN | 07-08-1989 | HAMMER |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument